# EUROPEAN PATENT APPLICATION

(11) **EP 4 659 742 A1**
(43) Date of publication of application: **10.12.2025**
(21) Application number: 24749814.0
(22) Date of filing: 31.01.2024
(51) Int. Cl.: A61K 9/20, A61K 31/437, A61P 1/04

(54) **PHARMACEUTICAL FORMULATION, PREPARATION METHOD THEREFOR, AND TABLET COMPOSITION**

(30) Priority: 01.02.2023 KR 20230013953
(71) Applicant: Jeil Pharmaceutical Co., Ltd., Seoul 06543 (KR); Onconic Therapeutics Inc., Gangnam-gu Seoul 06236 (KR)
(72) Inventor: LEE, Sibeum, Yongin-si Gyeonggi-do 17009 (KR); JEON, Soomin, Hwaseong-si Gyeonggi-do 18484 (KR); LEE, Dongho, Yongin-si Gyeonggi-do 17066 (KR); LEE, Youngeun, Suwon-si Gyeonggi-do 16512 (KR); PARK, Yejin, Hwaseong-si Gyeonggi-do 18478 (KR)
(74) Representative: Schön, Christoph
(86) International application number: PCT/IB2024/050870
(87) International publication number: WO 2024/161317

(57) **Abstract**

The present invention relates to a pharmaceutical formulation, a preparation method therefor and a tablet composition. The pharmaceutical formulation according to the present invention comprises an azetidin-1-yl{8-[(2,6- dimethylbenzyl)amino]-2,3-dimethylimidazo[1,2-a]pyridine-6-yl} methanone citrate salt as an active ingredient, and microcrystalline cellulose as an excipient.

## Description

### TECHNICAL FIELD

The present invention relates to a pharmaceutical formulation comprising imidazo[1,2-a]pyridine compounds or salts thereof, a preparation method therefor and a composition for tableting.

### BACKGROUND ART

In general, in order to be considered as a candidate for development as a pharmaceutical agent, a substance must have desirable biological properties as well as physical properties that enable its use pharmaceutically. However, even if a drug has excellent activity, it is often difficult to apply it to industrialization due to its poor formulation stability.

Meanwhile, imidazo[1,2-a]pyridine compounds or pharmaceutically acceptable salts thereof are pharmaceutical raw materials for preventing or treating gastrointestinal inflammatory diseases or gastric acid-related diseases, such as peptic ulcer, gastroduodenal ulcer, gastritis, gastroesophageal reflux disease (GERD), non-erosive reflux disease (NERD) and the like.

Therefore, the present inventors have made efforts to develop a stable pharmaceutical formulation for imidazo[1,2-a]pyridine compounds or pharmaceutically acceptable salts thereof, and have now completed the present invention.

### [Prior Documents]

### [Patent Document]

(Patent Document 1) Korean Patent No. 10-1777971

### DISCLOSURE OF INVENTION

### TECHNICAL PROBLEM

The purpose of the present invention is to provide a pharmaceutical formulation comprising imidazo[1,2-a]pyridine compound or a pharmaceutically acceptable salt thereof as an active ingredient.

Another purpose of the present invention is to provide a method for preparing the pharmaceutical formulation.

Still another purpose of the present invention is to provide a composition for tableting.

### SOLUTION TO PROBLEM

A pharmaceutical formulation for one purpose of the present invention comprises azetidin-1-yl{8-[(2,6-dimethylbenzyl)amino]-2,3-dimethylimidazo[1,2-a]pyridin-6-yl}methanone citrate salt as an active ingredient; and microcrystalline cellulose as an excipient.

In the present invention, azetidin-1-yl{8-[(2,6-dimethylbenzyl)amino]-2,3-dimethylimidazo[1,2-a]pyridin-6-yl}methanone is Compound 1 represented by Formula 1 below.

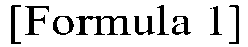

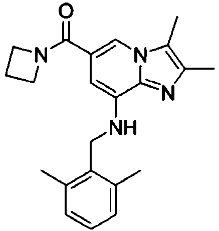

The active ingredient in the present invention is the citrate salt of Compound 1 represented by Formula 1, and hereinafter "azetidin-1-yl{8-[(2,6-dimethylbenzyl)amino]-2,3-dimethylimidazo[1,2-a] pyridin-6-yl}methanone citrate salt" and "citrate salt of Compound 1" are intended to refer to the same agent.

The citrate salt of Compound 1 can be represented by Formula 2 below. 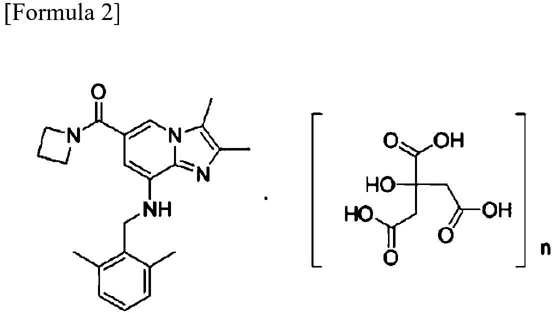 in Formula 2 above, n may be from 0.3 to 1.3, for example n may be from 0.5 to 1.

The citrate salt of Compound 1-which is the active ingredient of the present invention-is a drug that exhibits significantly superior bioavailability compared to the free base Compound 1 and is highly effective in the prevention or treatment of gastrointestinal inflammatory diseases or gastric acid-related diseases. Specifically, when the citrate salt of Compound 1 is administered orally, it reaches peak blood concentrations faster than the free base Compound 1, with Cmax that is at least 11-fold higher and AUC that is at least 5-fold higher.

Pharmaco-physically, the citrate salt of Compound 1 is in powder form and has very high scattering properties and even sticky property. Due to these properties, at least part of the powder of the citrate salt of Compound 1 easily forms agglomerates and is easily scattered irregularly even in a fine flow of air, thereby making the handling of the citrate salt of Compound 1 very difficult. This is very different from the powder properties of the solid phase of Compound 1 (free base). Therefore, even though the citrate salt of Compound 1 is a drug with better bioavailability than Compound 1, it is very difficult to formulate to have good tabletability while having pharmaceutically adequate flowability.

According to the present invention, it is possible to stably formulate the citrate salt of Compound 1 while preventing the dissolution rate of the citrate salt of Compound 1 from decreasing. In addition, the pharmaceutical formulation according to the present invention can be prepared in a stable shape, and when prepared on a large scale, the active ingredient contained in each of the pharmaceutical formulations can be uniformly contained per unit formulation. Furthermore, the pharmaceutical formulation is physically stable due to low friability or abrasion, and chemically stable without changes in temperature and/or humidity and long-term storage. Furthermore, the pharmaceutical formulation has excellent tabletability, so that tableting failures do not occur even when using a direct compression. Furthermore, the dissolution stability of the citrate salt of Compound 1 can be improved.

In one embodiment, the particle size D₉₀ of the citrate salt of Compound 1 of the pharmaceutical formulation according to the present invention may be 60 µm or less, for example 20 to 60 µm.

In one embodiment, in the pharmaceutical formulation according to the present invention, the weight ratio of citrate salt of Compound 1 to microcrystalline cellulose may be 1 : 3.5 to 1 : 12.

Accordingly, the citrate salt of Compound 1 can be stably formulated with excipients, while ensuring that the active ingredient contained in each of the pharmaceutical formulations is uniformly contained per unit formulation and preventing a decrease of the dissolution rate of the active ingredient in the formulation.

In one embodiment, the weight ratio of the citrate salt of Compound 1 to microcrystalline cellulose may be 1 : 5 to 1 : 9.

In one embodiment, the weight ratio of citrate salt of Compound 1 to microcrystalline cellulose may be 1 : 6 to 1 : 7.

In one embodiment, the unit formulation, i.e., the citrate salt of Compound 1 may be comprised in an amount of 5 to 50 mg per unit tablet. In this case, the microcrystalline cellulose may be comprised in an amount of 17.5 mg to 600 mg.

In one embodiment, the unit formulation may comprise 10 to 40 mg of the citrate salt of Compound 1 and 35 mg to 480 mg of the microcrystalline cellulose.

In one embodiment, the unit formulation may comprise 20 mg of the citrate salt of Compound 1 and 70 mg to 240 mg of the microcrystalline cellulose.

In one embodiment, the unit formulation may comprise 20 mg of the citrate salt of Compound 1 and 100 mg to 180 mg of the microcrystalline cellulose.

In one embodiment, the unit formulation may comprise 20 mg of the citrate salt of Compound 1 and 130 mg to 140 mg of the microcrystalline cellulose.

In one embodiment, the pharmaceutical formulation may comprise anhydrous lactose as an excipient.

In one embodiment, the weight ratio of the citrate salt of Compound 1 to anhydrous lactose may be 1 : 1 to 1 : 3.

In one embodiment, the weight ratio of the citrate salt of Compound 1 to anhydrous lactose may be 1 : 1.5 to 1 : 2.5.

In one embodiment, the unit formulation may comprise 10 to 30 mg of the citrate salt of Compound 1 and 10 to 45 mg of the anhydrous lactose.

In one embodiment, the unit formulation may comprise 20 mg of the citrate salt of said Compound 1 and 35 to 45 mg the anhydrous lactose.

In one embodiment, the particle size D₅₀ of the anhydrous lactose in the pharmaceutical formulation may be 110 to 130 µm.

As used herein, "particle size Dx = Y (wherein X and Y are positive numbers)" means that when the particle size (diameter of particles) distribution is represented by a cumulative curve, the particle size is Y at the point where the particle size is X% (% is calculated based on number, volume or weight) cumulatively from zero. Thus, particle size D₁₀ may be defined as the particle size at the 10% point on the cumulative curve of the particle size distribution, particle size D₅₀ may be defined as the particle size at the 50% point on the cumulative curve of the particle size distribution, and particle size D₉₀ may be defined as the particle size at the 90% point on the cumulative curve of the particle size distribution. In addition, "particle size D_{X}" may vary depending on whether it represents a percentage of the total accumulated particles based on number, volume or weight. The particle size D_{X} measurement may be determined by a laser diffraction method that records the diameter of a particle by viewing the particle as a volume equivalent sphere. When the particle size distribution is measured by the laser diffraction method, the value of X in the particle size D_{X} represents a percentage calculated by volume averaging. Thus, the laser diffraction method is sensitive to the volume of the particle and provides a volume-averaged particle size, which can be considered a weight-averaged particle size if the density is constant. Measurement of the volume-averaged particle size distribution can be performed using commercially available measuring devices based on laser diffraction/scattering according to the Mie theory. For example, a Mastersizer laser diffraction device from Malvern Instruments can be used to measure the particle size.

In one embodiment, the particle size D₅₀ of the present invention may be an average particle size.

In one embodiment, the microcrystalline cellulose as an excipient in the present invention may comprise a first microcrystalline cellulose having a particle size D₅₀ of 150 to 210 µm and a second microcrystalline cellulose having a particle size D₅₀ of 230 to 270 µm.

In one embodiment, the particle size D₅₀ of the first microcrystalline cellulose may be 160 to 200 µm, or 170 to 190 µm. In this case, the particle size D₅₀ of the second microcrystalline cellulose may be 240 to 260 µm.

In one embodiment, the first microcrystalline cellulose may be Vivapur^{®} 12, Avicel^{®} PH200 or the like; and the second microcrystalline cellulose may be Vivapur^{®} 200 or the like.

In one embodiment, the weight ratio of the first microcrystalline cellulose to the second microcrystalline cellulose may be 1 : 1 to 1 : 1.5.

By using the first microcrystalline cellulose and second microcrystalline cellulose together in the present invention, the excipient can be mixed very uniformly with the citrate salt of Compound 1 as the active ingredient, while obtaining excellent fluidity and tabletability of the mixture comprising the active ingredient and the excipient at the same time. Compared to the present invention, when the first microcrystalline cellulose or the second microcrystalline cellulose is used alone, or when microcrystalline cellulose having a particle size D₅₀ of 130 µm or less is used, it is difficult to achieve desirable formulation of tablets comprising the citrate salt of Compound 1 as the active ingredient with the mixture comprising the active ingredient and the excipient.

By including microcrystalline cellulose and/or anhydrous lactose satisfying the conditions described above as excipients for the citrate salt of Compound 1 in the present invention, the fluidity and tabletability of the composition for preparing pharmaceutical formulations can be improved, the active ingredient comprised in each of the pharmaceutical formulations can be uniformly contained per unit formulation, both the compressibility and disintegration of the pharmaceutical formulations can be improved, and the pharmaceutical formulations can be stable with respect to temperature and/or humidity.

In one embodiment, the pharmaceutical formulation may comprise, in addition to the active ingredient and excipients, pharmaceutically acceptable additives. Examples of the pharmaceutically acceptable additive include, but are not limited to, a binder, a disintegrating agent, a lubricant, a diluent, a pH adjuster, a solubilizing agent, a surfactant, a coating agent and the like, which may be utilized in two or more combinations.

In one embodiment, the pharmaceutical formulation may comprise, in addition to the active ingredient and excipients, a disintegrating agent and a lubricant.

Examples of the disintegrating agent may include starch or modified starch, such as sodium starch glycolate, corn starch, potato starch or pregelatinized starch; clay, such as bentonite, montmorillonite, or veegum; cellulose, such as hydroxypropyl cellulose or carboxymethylcellulose; alginates, such as sodium alginate or alginic acid; cross-linked celluloses, such as sodium croscarmellose; gums, such as guar gum or xanthan gum; cross-linked polymers, such as crospovidone; and effervescent agents, such as sodium bicarbonate, citric acid; and the like. Each of these may be utilized alone or in a mixture of two or more.

Examples of the lubricant may include calcium stearate, glyceryl monostearate, glyceryl palmitostearate, magnesium stearate, sodium lauryl sulfate, sodium stearyl fumarate, zinc stearate, stearic acid, hydrogenated vegetable oils, polyethylene glycol, sodium benzoate, talc, and the like. Each of these may be utilized alone or in a mixture of two or more.

In one embodiment, the lubricant of the present invention may comprise sodium stearyl fumarate and magnesium stearate. The pharmaceutical formulation of the present invention may have improved stability of the formulation and/or stability of the formulation due to the inclusion of sodium stearyl fumarate and magnesium stearate. In the pharmaceutical formulations of the invention, the weight ratio of sodium stearyl fumarate to magnesium stearate may be 5 : 2 to 1 : 3. For example, in the pharmaceutical formulation according to the present invention, when sodium stearyl fumarate is 100 parts by weight, magnesium stearate may be 40 to 300 parts by weight. In the pharmaceutical formulation according to the present invention, when the sodium stearyl fumarate is 100 parts by weight, the magnesium stearate may be 45 to 250 parts by weight.

Examples of the coating agent may include polyvinyl alcohol, hydroxypropylmethylcellulose, methylcellulose, ethylcellulose, polyvinyl acetate, polyethylene glycol, titanium dioxide, iron oxide, and the like; or the trade name Opadry^{®}, which include Opadry white, Opadry pink, Opadry green, Opadry orange, Opadry blue, Opadry yellow, Opadry brown, and the like. Each of these may be utilized alone or in a mixture of two or more.

In one embodiment, the pharmaceutical formulation may be an oral formulation.

In one embodiment, the pharmaceutical formulation is an oral formulation, which may be a tablet.

In one embodiment, the tablet may be a naked tablet or a coated tablet in which a naked tablet is coated. In this case, the naked tablet may comprise the active ingredient and excipients, and the coating layer may comprise a coating agent. As a coating agent, Opadry pink may be utilized.

In one embodiment, the pharmaceutical formulation may be a naked tablet comprising the active ingredient and microcrystalline cellulose.

In one embodiment, the pharmaceutical formulation may be a coated tablet wherein the naked tablet comprising the active ingredient and microcrystalline cellulose is coated by a coating layer.

The pharmaceutical formulation according to the present invention may comprise a composition for tableting, and the composition for tableting comprises the citrate salt of Compound 1 and microcrystalline cellulose. Since the components comprised in the composition for tableting are substantially the same as those described in the pharmaceutical formulation above, redundant detailed description will be omitted.

A method of preparing a pharmaceutical formulation according to the invention comprises:
preparing a composition for tableting by mixing the citrate salt of Compound 1 with microcrystalline cellulose; and
preparing a tablet by tableting the composition for tableting.

In preparing the composition for tableting, the citrate salt of Compound 1 may be mixed with microcrystalline cellulose in a weight ratio of 1 : 3.5 to 1 : 12.

In preparing the composition for tableting, anhydrous lactose may be mixed together.

The microcrystalline cellulose of the composition for tableting may comprise a first microcrystalline cellulose having a particle size D₅₀ of 150 to 210 µm and a second microcrystalline cellulose having a particle size D₅₀ of 230 to 270 µm.

The composition for tableting may further comprise a disintegrating agent and/or a lubricant.

The composition for tableting obtained as described above overcome both the scattering and sticky properties of the citrate salt of Compound 1, enabling the preparation of tablets with stable fluidity and good physical tabletability. The tablets prepared using the present composition are stable in shape, physically stable with low friability or abrasion, and chemically stable with respect to temperature and/or humidity and long-term storage. Furthermore, the citrate salt of Compound 1, which is the active ingredient in the tablet, has excellent dissolution rate and dissolution stability.

The step of preparing tablets may comprise the step of obtaining a naked tablet by tableting the composition for tableting. The step of obtaining a naked tablet may be carried out by direct compression.

The step of preparing tablets may comprise the steps of: tableting the composition for tableting to obtain a naked tablet; and coating the naked tablet to obtain a coated tablet.
(1) The pharmaceutical formulation according to the present invention comprises azetidine-1-yl{8-[(2,6-dimethylbenzyl)amino]-2,3-dimethylimidazo[1,2-a]pyridin-6-yl}methanone citrate salt as an active ingredient; and microcrystalline cellulose as an excipient.
(2) In the pharmaceutical formulation according to (1) above, the weight ratio of azetidine-1-yl{8-[(2,6-dimethylbenzyl)amino]-2,3-dimethylimidazo[1,2-a]pyridin-6-yl}methanone citrate salt to microcrystalline cellulose may be 1 : 3.5 to 1 : 12.
(3) In the pharmaceutical formulation according to (1) or (2) above, the azetidin-1-yl{8-[(2,6-dimethylbenzyl)amino]-2,3-dimethylimidazo[1,2-a]pyridin-6-yl}methanone citrate salt may be comprised in an amount of 5 to 50 mg per unit formulation.
(4) The pharmaceutical formulation according to any one of (1) to (3) above may comprise anhydrous lactose as an excipient.
(5) In the pharmaceutical formulation according to any one of (1) to (4) above, the microcrystalline cellulose may comprise a first microcrystalline cellulose having a particle size D₅₀ of 150 to 210 µm and a second microcrystalline cellulose having a particle size D₅₀ of 230 to 270 µm.
(6) In (5) above, the weight ratio of the first microcrystalline cellulose to the second microcrystalline cellulose may be 1 : 1 to 1 : 1.5.
(7) The pharmaceutical formulation according to any one of (1) to (6) above may comprise sodium stearyl fumarate and magnesium stearate as lubricants.
(8) In (7) above, the weight ratio of sodium stearyl fumarate to magnesium stearate may be 5 : 2 to 1 : 3.
(9) The pharmaceutical formulation according to any one of (1) to (8) above may be a tablet.
(10) The pharmaceutical preparation according to any one of claims (1) to (9) above may be a compressed material prepared by direct compression.
(11) The method for preparing the pharmaceutical formulation according to the present invention comprises: preparing a composition for tableting comprising azetidine-1-yl{8-[(2,6-dimethylbenzyl)amino]-2,3-dimethylimidazo[1,2-a]pyridin-6-yl}methanone citrate salt as an active ingredient; and microcrystalline cellulose as an excipient; and preparing a tablet by tableting the composition for tableting by direct compression.
(12) The pharmaceutical formulation according to any one of (1) to (10) above may be for the prevention or treatment of gastrointestinal inflammatory disease or gastric acid-related disease.
(13) The present invention provides use of a composition comprising azetidine-1-yl{8-[(2,6-dimethylbenzyl)amino]-2,3-dimethylimidazo[1,2-a]pyridin-6-yl}methanone citrate salt as an active ingredient; and microcrystalline cellulose as an excipient in the manufacture of the pharmaceutical formulation according to any one of (1) to (10) above which is for the prevention or treatment of gastrointestinal inflammatory disease or gastric acid-related disease.
(14) The present invention provides a method for preventing or treating gastrointestinal inflammatory disease or a gastric acid-related disease comprising administering to a subject the pharmaceutical formulation according to any one of (1) to (10) above.
(15) The present invention provides use of the pharmaceutical formulation according to any one of (1) to (10) above for preventing or treating gastrointestinal inflammatory disease or gastric acid-related disease.

### EFFECTS OF INVENTION

In the pharmaceutical formulation, the preparation method therefor and the composition for tableting according to the present invention, the pharmaceutical formulation can stably formulate azetidine-1-yl{8-[(2,6-dimethylbenzyl)amino]-2,3-dimethylimidazo[1,2-a]pyridin-6-yl}methanone citrate salt while preventing its dissolution rate from decreasing. In addition, the pharmaceutical formulation according to the present invention can be prepared in a stable shape, and when prepared on a large scale, the active ingredient comprised in each of the pharmaceutical formulations can be uniformly contained per unit formulation. Furthermore, the pharmaceutical formulation is physically stable with low friability/abrasion, and chemically stable with no changes in temperature and/or humidity and long-term storage. Furthermore, the dissolution rate and dissolution stability of azetidine-1-yl{8-[(2,6-dimethylbenzyl)amino]-2,3-dimethylimidazo[1,2-a]pyridin-6-yl}methanone citrate salt are excellent.

Accordingly, the oral pharmaceutical preparation comprising azetidine-1-yl{8-[(2,6-dimethylbenzyl)amino]-2,3-dimethylimidazo[1,2-a]pyridin-6-yl}methanone citrate salt as the active ingredient, which have remarkably excellent bioavailability, can also be highly effective in preventing or treating gastrointestinal inflammatory disease or gastric acid-related disease.

### BRIEF DESCRIPTION OF DRAWINGS

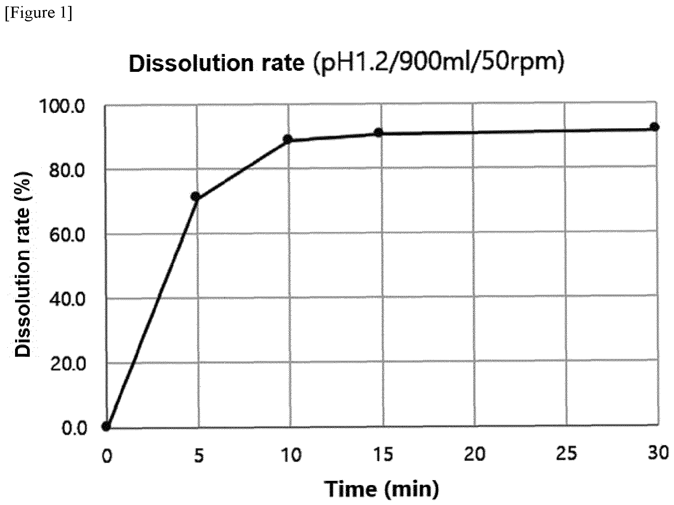

Figure 1 is a graph showing the dissolution properties over time of the tablet according to Example 1 of the present invention.

### MODE FOR THE INVENTION

Hereinafter, embodiments of the present invention will be described in detail. The following embodiments are illustrative of the invention, and the invention is not limited by these embodiments.

### Preparation Example 1: Preparation of Citrate Salt of Compound 1

Azetidin-1-yl{8-[(2,6-dimethylbenzyl)amino]-2,3-dimethylimidazo[1,2-a]pyridin-6-yl}methanone citrate salt was obtained according to the following process. Specifically, azetidin-1-yl{8-[(2,6-dimethylbenzyl)amino]-2,3-dimethylimidazo[1,2-a]pyridin-6-yl}methanone was obtained as described in Korean Patent No. 10-1777971. The NMR analysis of the azetidin-1-yl{8-[(2,6-dimethylbenzyl)amino]-2,3-dimethylimidazo[1,2-a]pyridin-6-yl}methanone obtained above is shown below.

¹H NMR (400 MHz, CDCl3); *δ* 7.63 (d, J=1.2 Hz, 1H), 7.13 (dd, J =8.4, 6.8 Hz, 1H), 7.06-7.04 (m, 2H), 6.42 (d, J= 1.2 Hz, 1H), 4.86-4.84 (m, 1H), 4.41-4.28 (m, 4H), 4.37 (d, J =4.4 Hz, 2H), 3.75-3.69 (m, 1H), 2.43-2.34 (m, 13H).

Then, the azetidin-1-yl{8-[(2,6-dimethylbenzyl)amino]-2,3-dimethylimidazo[1,2-a]pyridin-6-yl}methanone obtained above was mixed with an alcohol solvent (isopropyl alcohol, IPA), stirred and vacuum dried at about 30°C to 35°C to obtain a dry product. About 10 g of the obtained dry product was taken and stirred with about 167 g of acetone. To this, a solution of citric acid (about 5 g) dissolved in acetone (about 33 g) was added slowly over 60 minutes and stirred at the same temperature for 1 hour. The reaction mixture was cooled to about 20°C to 25°C and stirred for additional 1 hour, and the resulting solid was filtered, washed with acetone, and vacuum dried to obtain azetidin-1-yl{8-[(2,6-dimethylbenzyl)amino]-2,3-dimethylimidazo[1,2-a]pyridin-6-yl}methanone citrate salt. The NMR analysis results of the azetidin-1-yl{8-[(2,6-dimethylbenzyl)amino]-2,3-dimethylimidazo[1,2-a]pyridin-6-yl}methanone citrate salt obtained above are as follows.

¹H NMR (400 MHz, MeOD); *δ* 7.90 (s, 1H), 7.06-7.15 (m, 3H), 6.77 (s, 1H), 4.50 (t, J=7.2 Hz, 2H), 4.45(s, 2H), 4.24(t, J=7.2Hz, 2H), 2.80(d, J=15.6Hz, 2H), 2.70(d, J=12.0, 2H), 2.39-2.44(m, 11H), 2.35(s, 3H).

### Examples 1-29: Preparation of Tablets

A mixture was obtained by weighing the azetidin-1-yl{8-[(2,6-dimethylbenzyl)amino]-2,3-dimethylimidazo[1,2-a]pyridin-6-yl}methanone citrate salt (citrate salt of Compound 1) obtained according to Preparation Example 1 above and the components having an amount according to Table 1 to Table 6 below so that each component per unit tablet has the content of Table 1 to Table 6. The above mixture was subjected to direct compression to obtain tablets. The tableting pressure was 500 to 600 kgf in the direct compression. The tablets were then coated using Opadry.

In the present invention, a first microcrystalline cellulose having D₅₀ of about 180 µm and a second microcrystalline cellulose having D₅₀ of about 250 µm were used as raw materials for the tablets. In the case of anhydrous lactose, D₅₀ of about 121 µm was used.

**[Table 1]**

| **Raw material name** | **Content (mg/tablet)** | | | | |
|---|---|---|---|---|---|
| | **Example 1** | **Example 2** | **Example 3** | **Example 4** | **Example 5** |
| Citrate salt of Compound 1 | 20.0 | 20.0 | 20.0 | 20.0 | 20.0 |
| First microcrystalline cellulose | 62.0 | 66.7 | 53.4 | 62.0 | 62.0 |
| Sodium Stearyl Fumarate | 6.4 | 6.4 | 6.4 | 7.1 | 5.3 |
| Second microcrystalline cellulose | 71.4 | 66.7 | 80.0 | 71.4 | 71.4 |
| Anhydrous Lactose | 40.0 | 40.0 | 40.0 | 40.0 | 40.0 |
| Croscarmellose Sodium | 6.0 | 6.0 | 6.0 | 6.0 | 6.0 |
| Magnesium stearate | 4.2 | 4.2 | 4.2 | 3.5 | 5.3 |
| Opadry 03B54445 Pink | 8.0 | 8.0 | 8.0 | 8.0 | 8.0 |
| Total tablet content | 218.0 | 218.0 | 218.0 | 218.0 | 218.0 |

**[Table 2]**

| **Raw material name** | **Content (mg/tablet)** | | | | |
|---|---|---|---|---|---|
| | **Example 6** | **Example 7** | **Example 8** | **Example 9** | **Example 10** |
| Citrate salt of Compound 1 | 20.0 | 20.0 | 20.0 | 20.0 | 20.0 |
| First microcrystalline cellulose | 46.5 | 79.0 | 97.6 | 62.0 | 62.0 |
| Sodium Stearyl Fumarate | 6.4 | 6.4 | 6.4 | 7.0 | 6.2 |
| Second microcrystalline cellulose | 53.5 | 91.0 | 112.4 | 71.4 | 71.0 |
| Anhydrous Lactose | 40.0 | 40.0 | 40.0 | 40.0 | 38.0 |
| Croscarmellose Sodium | 6.0 | 6.0 | 6.0 | 6.0 | 5.9 |
| Magnesium stearate | 4.2 | 4.2 | 4.2 | 3.5 | 4.0 |
| Opadry 03B54445 Pink | 8.0 | 8.0 | 8.0 | 8.0 | 8.0 |
| Total tablet content | 184.6 | 254.6 | 294.6 | 217.9 | 215.1 |

**[Table 3]**

| **Raw material name** | **Content (mg/tablet)** | | | | |
|---|---|---|---|---|---|
| | **Example 11** | **Example 12** | **Example 13** | **Example 14** | **Example 15** |
| Citrate salt of Compound 1 | 20.0 | 20.0 | 20.0 | 20.0 | 20.0 |
| First microcrystalline cellulose | 64.0 | 67.0 | 55.0 | 58.0 | 62.0 |
| Sodium Stearyl Fumarate | 6.4 | 6.6 | 6.8 | 6.0 | 6.2 |
| Second microcrystalline cellulose | 69.0 | 67.0 | 82.5 | 80.0 | 68.0 |
| Anhydrous Lactose | 40.0 | 42.0 | 44.0 | 40.0 | 42.0 |
| Croscarmellose Sodium | 6.1 | 6.3 | 5.7 | 5.9 | 6.1 |
| Magnesium stearate | 4.1 | 4.2 | 4.3 | 4.4 | 4.0 |
| Opadry 03B54445 Pink | 8.0 | 8.0 | 8.0 | 8.0 | 8.0 |
| Total tablet content | 217.6 | 221.1 | 226.3 | 222.3 | 216.3 |

**[Table 4]**

| **Raw material name** | **Content (mg/tablet)** | | | | |
|---|---|---|---|---|---|
| | **Example 16** | **Example 17** | **Example 18** | **Example 19** | **Example 20** |
| Citrate salt of Compound 1 | 20.0 | 20.0 | 10.0 | 30.0 | 40.0 |
| First microcrystalline cellulose | 62.0 | 62.0 | 31.0 | 93.0 | 124.0 |
| Sodium Stearyl Fumarate | 6.4 | 6.6 | 3.2 | 9.6 | 12.8 |
| Second microcrystalline cellulose | 75.0 | 78.0 | 35.7 | 107.1 | 142.8 |
| Anhydrous Lactose | 44.0 | 38.0 | 20.0 | 60.0 | 80.0 |
| Croscarmellose Sodium | 6.3 | 6.0 | 3.0 | 9.0 | 12.0 |
| Magnesium stearate | 4.2 | 4.4 | 2.1 | 6.3 | 8.4 |
| Opadry 03B54445 Pink | 8.0 | 8.0 | 4.0 | 12.0 | 12.0 |
| Total tablet content | 225.9 | 223.0 | 109.0 | 327.0 | 432.0 |

**[Table 5]**

| **Raw material name** | **Content (mg/tablet)** | | | | |
|---|---|---|---|---|---|
| | **Example 21** | **Example 22** | **Example 23** | **Example 24** | **Example 25** |
| Citrate salt of Compound 1 | 5.0 | 5.0 | 10.0 | 10.0 | 30.0 |
| First microcrystalline cellulose | 15.5 | 28.0 | 31.0 | 48.0 | 93.0 |
| Sodium Stearyl Fumarate | 6.2 | 4.2 | 6.4 | 3.2 | 6.4 |
| Second microcrystalline cellulose | 17.9 | 32.0 | 35.7 | 72.0 | 107.1 |
| Anhydrous Lactose | 40.0 | 36.0 | 38.0 | 40.0 | 50.0 |
| Croscarmellose Sodium | 3.0 | 4.0 | 6.0 | 6.0 | 6.0 |
| Magnesium stearate | 4.0 | 4.2 | 4.2 | 2.1 | 4.2 |
| Opadry 03B54445 Pink | 4.0 | 6.0 | 8.0 | 8.0 | 8.0 |
| Total tablet content | 95.6 | 119.4 | 139.3 | 189.3 | 304.7 |

**[Table 6]**

| **Raw material name** | **Content (mg/tablet)** | | | | |
|---|---|---|---|---|---|
| | **Example 26** | **Example 27** | **Example 28** | **Example 29** | **Example 30** |
| Citrate salt of Compound 1 | 30.0 | 40.0 | 40.0 | 50.0 | 20.0 |
| First microcrystalline cellulose | 62.0 | 80.0 | 62.0 | 80.0 | 54.6 |
| Sodium Stearyl Fumarate | 8.2 | 6.4 | 6.4 | 6.4 | 2.3 |
| Second microcrystalline cellulose | 71.4 | 94.0 | 78.0 | 96.0 | 81.9 |
| Anhydrous Lactose | 40.0 | 60.0 | 40.0 | 40.0 | 40.0 |
| Croscarmellose Sodium | 6.0 | 6.0 | 6.0 | 6.0 | 6.0 |
| Magnesium stearate | 4.2 | 4.2 | 4.2 | 4.2 | 5.2 |
| Opadry 03B54445 Pink | 8.0 | 8.0 | 8.0 | 8.0 | 8.0 |
| Total tablet content | 229.8 | 298.6 | 244.6 | 290.6 | 218.0 |

### Comparative Example: Preparation of tablets

A comparative tablet composition capable of preparing tablets having the ingredients and contents of Table 7 which are the same as those of Table 1 was prepared, except that 20 mg of Compound 1 was used instead of the citrate salt of Compound 1.

In addition, a comparative tablet composition capable of preparing tablets having the ingredients and contents of Table 7 which are the same as those of Table 1 was prepared, except that 20 mg of the fumarate salt of Compound 1 was used instead of the citrate salt of Compound 1.

Each of the comparative tablet compositions was directly compressed to obtain tablets of Comparative Example 1 and Comparative Example 2. They were then coated using Opadry. In the direct compression process, it was confirmed that the tablets of Comparative Example 1 and Comparative Example 2 could not be stably compressed with substantially the same tableting pressure as the tablets according to Example 1, and comparative tablets were prepared by applying the tableting pressure of at least 1000 kgf, more specifically at least 1100 to 1200 kgf.

**[Table 7]**

| **Raw material name** | **Content (mg/tablet)** | |
|---|---|---|
| | **Comparative Example 1** | **Comparative Example 2** |
| Compound 1 | 20.0 | - |
| Fumarate of Compound 1 | - | 20.0 |
| First microcrystalline cellulose | 62.0 | 62.0 |
| Sodium Stearyl Fumarate | 6.4 | 6.4 |
| Second microcrystalline cellulose | 71.4 | 71.4 |
| Anhydrous lactose | 40.0 | 40.0 |
| Croscarmellose Sodium | 6.0 | 6.0 |
| Magnesium stearate | 4.2 | 4.2 |
| Opadry 03B54445 Pink | 8.0 | 8.0 |
| Total tablet content | 218.0 | 218.0 |

### E valuation 1: Dissolution Properties

Coated tablets obtained according to Example 1 were subjected to a time-dependent active ingredient dissolution test under pH 1.2 conditions. The dissolution test was performed according to the method described below, and the results are shown in Figure 1.

| | **pH 1.2 dissolution** |
|---|---|
| **Dissolution method** | Paddle method, Method 2 of the Korean Pharmacopoeia |
| **Stirring speed** | 50 rpm |
| **Dissolution volume** | 900 mL |
| **Dissolution temperature** | 37±0.5°C |
| **Analysis method** | HPLC UV absorbance spectrophotometer (254 nm) |
| **Column** | C18 (4.6 x 150 mm, 5 µm) |
| **Mobile phase** | 20 mmol/L sodium perchlorate and acetonitrile mixture (55:45 v/v, %) |

Referring to Figure 1, it can be confirmed that the tablets according to the present invention show about 80% or more dissolution of the citrate salt of Compound 1 within 10 minutes. This is a very good initial dissolution and confirms that the tablets according to the present invention exhibit a good initial dissolution rate.

### Evaluation 2: Accelerated condition

For each of the naked and coated tablets obtained through the same process as described in Example 1, the change in LOD (loss of dry weight, unit %) over 24 hours was determined after placing them in an accelerated chamber (40°C, 75%RH). The LOD measurement was performed according to the loss on drying test of the Korean Pharmacopoeia.

The LOD (%) was measured by packing the naked tablets according to the present invention in a polyethylene container with a lid together with 1 g of silica gel, and it was found that the measured value after 24 hours was almost no different from the initial value.

### Evaluation 3: Stress Condition

For each of the naked and coated tablets obtained through the same process as described in Example 1, the loss of dry weight (LOD) was measured for two weeks after placing them in harsh conditions (60°C, 80%RH). In addition, the content change, impurities change and dissolution properties under stress condition were also measured.

Each experiment was conducted according to the following.

| | **Content test** | **pH 1.2 dissolution** | **pH 4.0 dissolution** |
|---|---|---|---|
| **Diluted solution preparation** | Mix acetonitrile and water 60:40 (v/v, %) | - | |
| **Test solution preparation** | 5 tablets / 500 mL dilution → filtration through a 0.45 um nylon filter | - | |
| **Dissolution method** | - | Paddle method, Method 2 of the Korean Pharmacopoeia | |
| **Stirring speed** | - | 50 rpm | 75 rpm |
| **Dissolution volume** | - | 900 mL | |
| **Dissolution temperature** | - | 37±0.5°C | |
| **Analysis method** | HPLC UV absorbance spectrophotometer (254 nm) | | |
| **Columns** | C18 (4.6 × 150 mm, 5 µm) | | |
| **Moblie phase** | 20 mmol/L sodium perchlorate and acetonitrile mixture (55:45 v/v, %) | | |

When the naked tablets according to the present invention were packaged in polyethylene containers with lids (without silica gel) and the LOD (%) was measured, it was confirmed that the difference between the initial value and the measured value after 2 weeks was significantly lower compared to the naked tablets obtained according to Comparative Examples 1 and 2. In other words, the naked tablets according to the present invention are stable tablets with very low hygroscopicity. On the other hand, when the same LOD (%) was measured for the tablets containing Compound 1 or the fumarate salt of Compound 1, it was confirmed that the value increased significantly after 2 weeks.

Under stress condition, the results of the change in the content of the naked tablets according to Example 1 of the present invention are as follows.

**[Table 8]**

| **Initial** | **Stress 1w** | **Stress 2w** |
|---|---|---|
| | **Bottle** | **Bottle** |
| 99.3 | 98.8 | 99.6 |

Referring to Table 8 above, it can be known that there was no change in the content in each of the first and second weeks compared to the initial value. In other words, it can be confirmed that the naked tablets according to the present invention is very stable. In addition, even if the same content change is measured for the coating tablets according to the present invention, it can be confirmed that there is no change in the content at least until the second week of stress condition.

Under stress condition, the purity of the naked tablets according to the present invention was measured to check for the occurrence of impurities, and it was found that the purity remained substantially unchanged in the first and second weeks compared to the initial purity. In addition, the purity of the coated tablets according to the present invention was measured to check for the occurrence of impurities, and it was found that the purity remained substantially unchanged in the second and fourth weeks compared to the initial purity.

Furthermore, under stress condition, the change in dissolution rate was checked, and the dissolution rate was performed at pH 1.2 and pH 4.0. As a result, it was found that at pH 1.2 and pH 4.0, respectively, the dissolution rate of the naked tablets and coated tablets according to the present invention remained substantially unchanged and stable even at the first and second week of stress condition compared to the initial dissolution rate. For the naked tablets containing the fumarate salt of Compound 1, the change in dissolution rate was measured equally at pH 1.2 and pH 4.0 after storage under stress condition, and it was confirmed that the change in dissolution rate was significantly higher for the fumarate salt of Compound 1. Therefore, it can be confirmed that the tablets according to the present invention comprising the citrate salt of Compound 1 as the active ingredient exhibit not only excellent dissolution rate but also excellent dissolution stability.

### Evaluation 4: Evaluate content uniformity

Tablets (coated tablets) according to Example 1 were prepared according to the uniformity test method for coated tablets in the general test method. The content of citrate salt of Compound 1 for 10 tablets of them was measured, and the content uniformity was evaluated by converting it to a percentage of the initial content. The results are shown in Table 9.

**[Table 9]**

| **Sample Type** | **Content (%)** |
|---|---|
| **1** | 100.4 |
| **2** | 99.2 |
| **3** | 100.0 |
| **4** | 98.5 |
| **5** | 97.3 |
| **6** | 99.3 |
| **7** | 97.6 |
| **8** | 99.0 |
| **9** | 100.6 |
| **10** | 100.3 |

Referring to Table 9, it can be confirmed that the average value is 99.2% and the deviation is only 1.2%, i.e., the tablets (coated tablets) according to Example 1 have a very good content uniformity.

While the above has been described with reference to preferred embodiments of the invention, it would be understood by a skilled artisan that various modifications and changes can be made to the invention without departing from the idea and scope of the invention as recited in the claims below.

## Claims

1. A pharmaceutical formulation comprising azetidine-1-yl{8-[(2,6-dimethylbenzyl)amino]-2,3-dimethylimidazo[1,2-a]pyridin-6-yl}methanone citrate salt as an active ingredient; and microcrystalline cellulose as an excipient.

2. The pharmaceutical formulation according to Claim 1, wherein a weight ratio of azetidin-1-yl{8-[(2,6-dimethylbenzyl)amino]-2,3-dimethylimidazo[1,2-a]pyridin-6-yl}methanone citrate salt to microcrystalline cellulose is 1 : 3.5 to 1 : 12.

3. The pharmaceutical formulation according to Claim 1, wherein the azetidin-1-yl{8-[(2,6-dimethylbenzyl)amino]-2,3-dimethylimidazo[1,2-a]pyridin-6-yl}methanone citrate salt is comprised in an amount of 5 to 50 mg per unit formulation.

4. The pharmaceutical formulation according to Claim 1, which comprises anhydrous lactose as the excipient.

5. The pharmaceutical formulation according to Claim 1, wherein the microcrystalline cellulose comprises a first microcrystalline cellulose having a particle size D₅₀ of 150 to 210 µm and a second microcrystalline cellulose having a particle size D₅₀ of 230 to 270 µm.

6. The pharmaceutical formulation according to Claim 5, wherein a weight ratio of the first microcrystalline cellulose to the second microcrystalline cellulose is 1 : 1 to 1 : 1.5.

7. The pharmaceutical formulation according to Claim 1, which comprises sodium stearyl fumarate and magnesium stearate as lubricants.

8. The pharmaceutical formulation according to Claim 7, wherein a weight ratio of sodium stearyl fumarate to magnesium stearate is 5 : 2 to 1 : 3.

9. The pharmaceutical formulation according to Claim 1, which is a tablet.

10. The pharmaceutical formulation according to Claim 9, which is a compressed material prepared by direct compression.

11. A method for preparing a pharmaceutical formulation comprising:
preparing a composition for tableting comprising azetidine-1-yl{8-[(2,6-dimethylbenzyl)amino]-2,3-dimethylimidazo[1,2-a]pyridin-6-yl}methanone citrate salt as an active ingredient; and microcrystalline cellulose as an excipient; and
preparing a tablet by tableting the composition for tableting by direct compression.

12. The pharmaceutical formulation according to Claim 1, which is used for the prevention or treatment of gastrointestinal inflammatory disease or gastric acid-related disease.

13. Use of a composition comprising azetidine-1-yl{8-[(2,6-dimethylbenzyl)amino]-2,3-dimethylimidazo[1,2-a]pyridin-6-yl}methanone citrate salt as an active ingredient; and microcrystalline cellulose as an excipient in the manufacture of a pharmaceutical formulation as defined in any one of Claims 1 to 10 which is for the prevention or treatment of gastrointestinal inflammatory disease or gastric acid-related disease.

14. A method for preventing or treating gastrointestinal inflammatory disease or gastric acid-related disease, comprising administering to a subject a pharmaceutical formulation as defined in any one of Claims 1 to 10.

15. Use of a pharmaceutical formulation as defined in any one of Claims 1 to 10 for preventing or treating gastrointestinal inflammatory disease or gastric acid-related disease.
